# DEMANDE DE BREVET EUROPEEN

(11) **EP 2 156 822 A1**
(43) Date de publication de la demande: **24.02.2010**
(21) Numéro de dépôt: 08290784.1
(22) Date de dépôt: 19.08.2008
(51) Int. Cl.: A61K 8/97, A61Q 7/00

(54) **Composition végétale à usage capillaire externe et préparation huileuse comportant une telle composition**

(71) Demandeur: Znagui, Imad, 77340 Pontault Combault (FR); Znagui, Malika, 77340 Pontault Combault (FR)
(72) Inventeur: Znagui, Imad, 77340 Pontault Combault (FR); Znagui, Malika, 77340 Pontault Combault (FR)

(57) **Abrégé**

L'invention concerne une préparation médicinale végétale ancestrale nouvelle corrélée à un procédé à usage externe appliqué sur le cuir chevelu. Cette composition de forme huileuse associée à deux substances en poudre peut se présenter en cosmétique pour les soins des cheveux en crème, shampooing, lotion et masque. Cette préparation est destinée aux personnes hommes, femmes et enfants subissant la chute des cheveux ou une calvitie partielle, et souffrant de leurs conséquences sur le plan psychologique et esthétique. Cette composition s'adresse aussi aux personnes ayant tout type de cheveux : cheveux fragiles, gras, manquant de vitalité. Elle permet également de lutter contre les maladies dermiques comme l'allergie, l'eczéma, le psoriasis. L'invention repose sur cette préparation végétale à usage industriel simple possédant un nouveau stimulant naturel pour favoriser croissance des cheveux qui sera identifié par des tests cliniques. La spécificité du contenu de cette préparation et son procédé de combinaison sont liés aux dosages des quatre composants permettant de produire de manière synergique des agents actifs et toniques stimulant le cuir chevelu. Ainsi cette préparation végétale sans additif chimique permet de créer les conditions optimales à la croissance des cheveux à court et à long terme. Elle améliore aussi l'aspect esthétique des cheveux en leur donnant vitalité, volume et éclat.

## Description

La nouveauté de l'invention repose sur cette préparation végétale ancestrale à usage industriel simple possédant un nouveau stimulant naturel pour freiner la chute des cheveux et favoriser leur croissance. Elle est constituée de deux substances végétales en poudre associées à deux substances huileuses. La spécificité du contenu de cette préparation et son procédé de combinaison sont liés particulièrement aux quatre composants permettant de produire de manière synergique des agents actifs et toniques stimulant le cuir chevelu. Certes l'efficacité de cette composition a été démontrée par l'usage dans un cadre familial restreint depuis un siècle. A cet égard, Il est impératif de la breveter avant de la divulguer auprès du public, dermatologues et professionnels du cosmétique. Toutefois des tests cliniques sont prévus après le dépôt du brevet afin d'identifier les composants actifs de cette préparation de manière expérimentale. A noter que cette composition permet de créer de manière visible les conditions optimales à la croissance des cheveux à court et à long terme. Elle améliore l'aspect esthétique des cheveux en leur donnant vitalité,
volume et éclat. De même, il a été constaté que cette composition peut être à usage dermatologique contre certaines maladies du cuir chevelu comme les allergies, eczémas et psoriasis. Des tests sont prévus pour valider la composition classée en tant que médicament pour le cuir chevelu.

Actuellement, dans l'état de la technique, plusieurs préparations chimiques et autres traitements complexes, parfois douloureux et coûteux proposent des solutions variées pour freiner la chute des cheveux et favoriser la pousse des cheveux. Cependant, les résultats de ces traitements sont souvent moins ressentis positivement par les utilisateurs et ne sont pas visibles à court terme. De plus, ces préparations provoquent parfois des irritations du cuir chevelu. Par ces motifs, ces mêmes utilisateurs se lassent et arrêtent souvent le traitement.

L'invention concerne une préparation médicinale végétale nouvelle corrélée à un procédé exclusivement à usage externe appliqué sur le cuir chevelu. Cette composition de forme huileuse pourrait se présenter en crème, shampooing, lotion et masque capillaires. Cette préparation est destinée aux personnes hommes, femmes et enfants subissant la chute des cheveux ou une calvitie partielle, et souffrant de leurs conséquences sur le plan psychologique et esthétique. Cette composition s'adresse aussi aux personnes ayant tout type de cheveux : cheveux fragiles, gras, secs et manquant de vitalité.

L'invention repose sur cette composition qui permet, à court et à long terme, d'apporter des solutions visibles et durables au niveau de la régénération du cuir chevelu. Cette préparation est constituée de quatre composants végétaux produisant des agents actifs, stimulant le cuir chevelu, freinant, dès le premier mois, la chute des cheveux, puis arrêtant de manière effective cette chute à partir du second mois et plus. Par ces motifs, la composition est un stimulant naturel des fibres kératines favorisant une repousse progressive et régulière des cheveux dès la 10^{ème} semaine.

A ce titre, l'invention concerne l'effet synergique des quatre composants de la préparation et du procédé de combinaison de deux extraits végétaux en poudre et de deux extraits végétaux huileux sur le cuir chevelu. Cette composition dynamise les fibres kératines, freine la chute des cheveux, fixe les cheveux à la racine après la troisième utilisation et favorise une croissance progressive des cheveux après deux mois d'utilisation.

Depuis un siècle à ce jour, aucun effet secondaire n'a été observé sur les utilisateurs de la préparation adultes et enfants appartenant à un cadre familial restreint. A ce titre, le certificat de conformité de ce produit sur le plan dermique a été délivré par le laboratoire Cosmepar, France qui a appliqué le test dermique sur 20 sujets. Ci-joint le certificat de conformité (Document1, document2). Il convient de préciser qu'en l'état actuel de la technique, nous constatons que les performances bénéfiques de cette composition sont étroitement liées au mélange et aux dosages des quatre composants, non divulgués au public à ce jour. (Document 3) Par conséquent, il est envisageable d'effectuer des recherches sur le dosage de chaque composant inclus dans cette préparation médicinale. De plus, il est envisageable de proposer cette composition sous plusieurs formes autres que celle présentée dans notre requête actuelle, en crème, lotion, shampooing pour cheveux.

Outre les effets de stimulation des fibres kératines et une fixation des cheveux à la racine, les utilisateurs ressentent dès la troisième application un changement visible au niveau de l'aspect esthétique des cheveux : cheveux plus éclatants, plus soyeux et plus volumineux, plus faciles à coiffer. Par ces motifs, cette préparation médicinale apporte des solutions sur le plan cosmétique en particulier au niveau de l'entretien et des soins des cheveux. (Document 4, document 5).

En ce qui concerne la fabrication industrielle de la préparation, la composition se rapporte à une combinaison (mélange) simple de quatre substances d'extraits végétaux se présentant dans un flacon avec les dosages suivants ou en dosettes de 18ml chacune.

### Composition du produit

| Nom INC des ingrédients | Classement | % d'utilisation Dans le produit fini |
|---|---|---|
| RUTA GRAVEOLENS EXTRACT (A) | 10 | 7,5% |
| EUGENIA CARYOPHYLLUS LEAF (B) | 10 | 7,5% |
| OLEA EUROPAEA OIL = C | 65 | 70% |
| ARGANIA SPINOSA OIL = D | 15 | 15% |

| | | |
|---|---|---|
| **A = Poudre** fine et homogène issue du broyage des grains de la plante **PEGANUM HARMALA,** le poids utilisé est de **8gr / 7% du poids de la composition qui est de 106 gr** **B = Poudre** fine et homogène issue du broyage des **CLOUS DE GIROFLE,** le poids utilisé est de **8gr. 7% du poids de la composition qui est de 106 gr** **C = Huile d'olives,** la quantité utilisée est de **90ml.** / 70% du poids de la composition qui est de 106gr **D = Huile d'ARGAN,** la quantité utilisée est de **20ml. /15% du poids de la composition qui est de 106gr.** La composition est constituée de 110ml de substance huileuse et 16gr de substance en poudre. Cette préparation est schématisée de la manière suivante : **A + B + C + D = E** **E = Nouvel agent stimulant du cuir chevelu freinant la chute des cheveux favorise la croissance et les soins des cheveux et guérit les maladies dermiques du cuir chevelu.** | | |

La mise en oeuvre de cette technique et les effets synergiques de cette préparation montrent que tous les composants sont indissociables créant un agent dynamisant le cuir chevelu et la croissance des cheveux. Dans cette optique, l'invention ne se limitera pas au dosage de chaque composant précédemment décrit, il est envisageable de varier ces doses selon le concept et la finalité de cette composition. De même, il est envisageable de proposer cette préparation sous plusieurs formes : en crème, shampooing, masque, lotion capillaire afin de faciliter l'usage de celle-ci aux utilisateurs.

Par ailleurs, nous avons observé que la substance huileuse constituée de deux huiles d'OLIVE et d'ARGAN en tant que conservateurs naturels permet de favoriser la pénétration dans les fibres kératines des deux substances en poudre de PEGANUM HARMALA et de CLOUS DE GIROFLE et régénérant le cuir chevelu et le protégeant contre le vieillissement.
De plus, selon les utilisateurs, cette substance huileuse associée aux deux composants en poudre redonne de la vitalité et de l'éclat aux cheveux après la troisième application : cheveux plus soyeux, plus beaux, plus volumineux, plus faciles à coiffer.

En ce qui concerne la forme de cette préparation médicinale, elle se présente en substance huileuse dans deux flacons : chaque flacon contient 110ml pour une durée d'un mois avec deux applications par semaine, et ce, afin d'obtenir des résultats accélérés pour freiner la chute des cheveux et régénérer le cuir chevelu. Les performances de cette composition sont décrites par le biais des phases suivantes :
- une phase de renforcement du cuir chevelu : fixation des cheveux à la racine et freinage de la chute des cheveux après la troisième utilisation de la préparation. Cette phase est accompagnée d'une amélioration de l'aspect des cheveux plus éclatants, plus volumineux, plus facile à coiffer.
- une phase de régénération du cuir chevelu accompagnée d'une croissance progressive des cheveux avec arrêt de chute des cheveux après la 7^{ème} utilisation.
- une phase de croissance des cheveux : une pousse régulière des cheveux au niveau des entrées, accompagnée d'un aspect plus beau, plus volumineux des cheveux après la 16 utilisation qui correspond à deux mois et plus. Une pousse circulaire au niveau de la tonsure a été constatée chez les adultes masculins.(Document 5bis)photo)
- un effet visible de guérison pour l'allergie, l'eczéma et psoriasis du cuir chevelu après la 3^{ème} utilisation de la composition..
En résumé, l'efficacité et les résultats bénéfiques de cette préparation cités ci-dessus sur le cuir chevelu des utilisateurs sont observés de manière visible durant deux mois avec une application ou deux applications par semaine pour des résultats accélérés. C'est pourquoi la composition huileuse sera présentée sous forme de deux flacons de 110ml pour une durée de deux mois. Après, l'utilisateur pourra utiliser cette préparation à long terme une fois les deux semaines pour se protéger contre le vieillissement du cuir chevelu, la calvitie progressive et assurer des soins pour ses cheveux.

En ce qui concerne le mode d'emploi de la préparation, il est conseillé de suivre la procédure suivante afin d'obtenir les résultats attendus de cette composition sur le cuir chevelu :
- Secouer plusieurs fois le contenu du flacon fermé afin de diluer la substance solide dans le composant huileux de façon homogène. Si le produit est présenté en dosettes de 18ml chacune, l'utilisateur n'aura pas à procéder ainsi.
- Mettre dans un récipient une petite quantité de la préparation et tremper un bout de coton dans celle-ci.
- Répartir en massant zone par zone l'ensemble du cuir chevelu.
   - Masser le reste des cheveux avec ce qui reste de substance huileuse.
- Laisser se reposer le produit sur les cheveux durant 4 heures ou de préférence, toute la nuit selon le mode artisanal, dans la mesure où les fibres kératines sont plus réceptives.
- Le matin, dégraisser les cheveux avec du shampoing à base d'argile, recommandé pour les cheveux longs ou utiliser votre shampooing habituel. A noter que cette composition odoriférante naturelle ne gêne pas les utilisateurs. De même cette préparation ne provoque aucune irritation au niveau du cuir chevelu.

Afin de confirmer la nouveauté de la composition sur le plan international et en réponse au rapport préliminaire de l'INPI de France concernant les deux brevets cités, ci-joint le rapport de recherche et les arguments relatés pour valider l'originalité de cette préparation végétale huileuse.(Documents 6 à 15)

### I Le document FR 2696640 A1 du 15/04/ 1994

Le document cite une composition arrêtant la chute des cheveux constituée de 8 éléments dans lesquels se trouve le clou de girofle avec une dose infime de 5%. Certes cette substance est incluse dans notre composition (huile d'ARGAN, huile d'OLIVES, poudre de CLOUS DE GIROFLE et poudre de grains de PEGANUM HARMALA), mais nous constatons que le document FR 2696640 A1 du 15/04/ 1994 ne cite en aucune manière le clou de girofle dans une préparation telle qu'elle est citée ou présentée dans nos revendications 1, 2 et 3 ci-joint :
1) Composition à usage capillaire externe permettant de stimuler, régénérer, entretenir le cuir chevelu caractérisée en ce qu'elle comprend quatre composants indissociables à effet synergique : huile d'ARGAN, huiled'OLIVES, poudredeCLOUS DE GIROFLE
   et poudre de grains de PEGANUM HARMALA.
2) Composition selon la revendication 1 caractérisée en ce qu'elle comprend une poudre fine et homogène issue du broyage des grains de la plante PEGANUM HARMALA dont le poids est de 8gr , une poudre fine et homogène de CLOUS DE GIROFLE dont le poids est de 8gr, l'Huile d'olives dont la quantité utilisée est de 90ml, Huile d'ARGAN dont la quantité utilisée est de 20ml.
3) Composition capillaire selon les revendications 1 et 2 caractérisée par le fait que le dosage proposé de chaque composant peut varier selon le concept de cette préparation qui repose sur la combinaison synergique des quatre éléments de celle-ci.
   Par ailleurs, la fabrication de la préparation du document FR 2696640 A1 du 15/04/ 1994 est très complexe (page 2 : ligne 1 à la ligne 14) pour obtenir un onguent ou une pommade, ce qui ne ne correspond pas du tout à la nature de notre composition qui est huileuse et qui diffère au niveau de l'application et de la méthode.
Par ces motifs, nous revendiquons l'originalité de notre composition par rapport à ce document (brevet) cité pertinent dans le rapport préliminaire et nous maintenons nos revendications 1, 2, 3, 4, 5.

### II Le document 240244A1 du 19/03/2004

Le document cite un complexe de 67 substances constituées de plantes (trois groupes: des fleurs, des graines et des écorces) et de bio-énergisant dans le lequel est cité l'huile d'argan de manière non quantifiée « une quantité efficace ou un pourcentage de l'huile d'argan est utilisée pour sa forte concentration », et ce, parmi plus d'une soixante de composants » page 6. De même, ce complexe d'actif végétal et de composantes chimiques cite également l'huile animale. A ce titre, certes, l'huile d'argan est incluse dans notre composition (huile d'ARGAN, huile d'OLIVES, poudre de CLOUS DE GIROFLE et poudre de grains de PEGANUM HARMALA), mais nous constatons que le document 2402444 A1 du 19/03/2004 ne cite en aucune manière l'huile d'argan dans une préparation telle qu'elle est citée ou présentée dans nos revendications 1, 2 et 3, 4, 5. De plus, les techniques de préparation complexe de l'actif végétal (pulvérisation, la fermentation : étape , étape 2, page 10) ne correspondent en aucune manière à notre méthode de préparation. Par ces motifs, nous revendiquons l'originalité de notre composition par rapport à ce document (brevet) 2402444 A1 du 19/03/2004 cité pertinent dans le rapport préliminaire et nous maintenons nos revendications 1, 2, 3, 4, 5.

### III Le paganum

Dans le rapport préliminaire, des articles divers et dictionnaires ont signalé généralement les effets potentiels de l'extrait de péganum sur d'autres aspects que la calvitie (bortifacient, alterative, aphrodisiac, digestive, diuretic, emmenagogue, galactogogue, halluconogénic, ophta. De même, il est ingurgité et non appliqué sur le cuir chevelu Certes, le péganum a été inclus dans notre composition (huile d'ARGAN, huile d'OLIVES, poudre de CLOUS DE GIROFLE et poudre de grains de PEGANUM HARMALA), mais aucun document ou brevet à notre connaissance ne le cite dans une composition telle qu'elle est citée ou présentée dans nos revendications 1, 2 et 3, 4, 5. Ses effets sur le plan dermique au niveau du cuir chevelu n'a pas été validé par des recherches cliniques. Or notre composition ancestrale contenait ce produit depuis une dizaine de générations et ne provoque aucune allergie en respectant une dose appropriée.
En conclusion, la composition huileuse qu'on nommé o!chaïmaniz apporte des solutions à la croissance des cheveux, améliore aussi l'aspect esthétique des cheveux : cheveux plus éclatants, plus soyeux, plus volumineux, plus facile à coiffer. De même, cette préparation a des effets bénéfiques contre les maladies du cuir chevelu. Par ces motifs, cette préparation est destinée à l'usage pharmaceutique et cosmétique dans sa composition naturelle et son parfum naturel sans additif chimique.

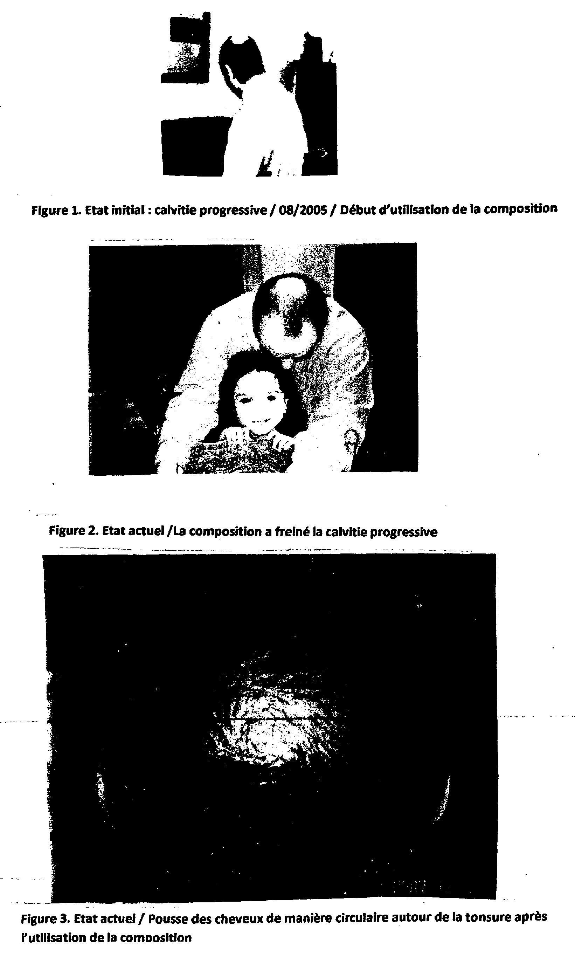

## Revendications

1. Composition à usage capillaire externe permettant de stimuler, régénérer, entretenir le cuir chevelu **caractérisée en ce qu'**elle comprend quatre composants indissociables à effet synergique : huile d'ARGAN, huile d'OLIVES, poudre de CLOUS DE GIROFLE et poudre de grains de PEGANUM HARMALA.

2. Composition selon la revendication 1 **caractérisée en ce qu'**elle comprend une poudre fine et homogène issue du broyage des grains de la plante PEGANUM HARMALA dont le poids est de 8gr , une poudre fine et homogène de CLOUS DE GIROFLE dont le poids est de 8gr, l'Huile d'olives dont la quantité utilisée est de 90ml, Huile d'ARGAN dont la quantité utilisée est de 20ml.

3. Composition capillaire selon les revendications 1 et 2 **caractérisée par**
**le fait que** le dosage proposé de chaque composant peut varier selon le concept de cette préparation qui repose sur la combinaison synergique des quatre éléments de celle-ci.

4. Composition capillaire selon la revendication 1 et 2 et 3 **caractérisée en ce qu'**elle comprend un composant huileux pouvant se présenter, selon le concept de cette préparation sous forme de crème, shampooing, lotion, masque pour les cheveux.

5. Composition capillaire selon les revendications 1, 2, 3, 4, **caractérisée en ce qu'**elle comprend des agents actifs pour le traitement pharmaceutique des cheveux et pour les soins des cheveux dans le secteur cosmétique qui seront identifiés de manière scientifiques par des tests cliniques après divulgation de la composition (après dépôt du brevet).

6. Composition capillaire en ce qu'elle comprend des agents actifs pour guérir des maladies du cuir chevelu à identifier par des tests cliniques après divulgation de la composition (après dépôt du brevet).
